# EUROPÄISCHE PATENTSCHRIFT

(11) **EP 1 732 475 B1**
(45) Veröffentlichungstag und Bekanntmachung des Hinweises auf die Patenterteilung: **23.06.2010**
(21) Anmeldenummer: 05742223.0
(22) Anmeldetag: 09.03.2005
(51) Int. Cl.: A61F 2/36

(54) **MODULARES GELENKPROTHESENSYSTEM**
MODULAR JOINT PROSTHESIS SYSTEM
SYSTEME DE PROTHESE D'ARTICULATION MODULAIRE

(30) Priorität: 17.03.2004 DE 102004013368
(43) Veröffentlichungstag der Anmeldung: 20.12.2006
(73) Patentinhaber: Merete Medical GmbH, 12247 Berlin (DE)
(72) Erfinder: ANAPLIOTIS, Emmanuell, 14195 Berlin (DE); KRANZ, Curt, 14163 Berlin (DE)
(74) Vertreter: Hannig, Wolf-Dieter
(86) Internationale Anmeldenummer: PCT/DE2005/000395
(87) Internationale Veröffentlichungsnummer: WO 2005/089676

(56) Entgegenhaltungen:
- EP-A- 0 457 222
- WO-A-01/82843
- DE-A1- 10 056 698
- DE-A1- 10 306 793
- DE-U1- 9 103 574
- FR-A- 2 854 320
- US-A- 5 405 394

## Beschreibung

Die Erfindung betrifft ein modulares Gelenkprothesensystem entsprechend dem Oberbegriff des Patentanspruchs 1.

Eine der artige Prothese ist aus dem deutschen Gebrauchsmuster DE 9103574 U bekannt. Bei dem BioBall® Steckkopf-System der Firma Merete (Katalog Bioball GR 02/2002) sind außerdem Zwischenelemente (Adapter) mit unterschiedlichen Längen vorgesehen.

Der Zweck einer solchen Prothese bzw. der betreffenden Zwischenelemente besteht darin, die Position von Gelenkpfanne und Gelenkkugel bei der Implantation oder einer Reoperation derart aufeinander abzustimmen, dass die resultierende Gelenkmechanik den beim betreffenden Patienten vorliegenden Gegebenheiten optimal angepasst ist.

Nachteilig bei den bekannten Prothesen ist, dass eine Vielzahl von unterschiedlichen Zwischenelementelementen vorrätig gehalten werden muss, um - ausgehend vom Femurschaft - die physiologisch korrekte Rotationsposition der Gelenkkugel in der Gelenkpfanne zu erzielen. Zwar kann ein Zwischenelement, bei dem der Innenkonus und der Außenkonus nicht koaxial fluchten zur optimalen Anpassung rotiert werden, die erreichbaren Positionen sind aber stets nur auf einem Kreisring gelegen.

Der Erfindung liegt die Aufgabe zugrunde, eine Prothese der eingangs angegebenen Gattung anzugeben, welche den genannten Nachteil nicht aufweist und es gestattet, die Geometrie des Gelenks für einen breiten Verwendungsbereich unter Benutzung einer Auswahl von nur wenigen Zwischenelementteilen auszugleichen.

Diese Aufgabe wird mit den im kennzeichnenden Teil des Anspruchs 1 angegebenen Maßnahmen gelöst.

Die Erfindung beruht dabei auf der Erkenntnis, dass anstelle eines Zwischenelements mit Achsenversatz zwei aufeinander abgestimmte Zwischenelemente gemeinsam eingesetzt werden. Die beiden Zwischenelemente weisen jeweils nicht koaxiale Außen- und Innenkonen auf, deren Achsen gleichsinnig radial zueinander versetzt und/oder abgewinkelt sind. Sie werden miteinander durch Zusammenstecken von Innen- und Außenkonus verbunden und können durch gegenseitiges Verdrehen relativ zueinander so positioniert werden, dass die Achsen des jetzt nach außen weisenden Paars von Außen- und Innenkonus in einem Bereich von koaxialer Fluchtung - oder nahezu koaxialer Fluchtung - bis zu einem maximalem Versatz bzw. einer maximaler Abwinklung verdrehbar sind. Die räumliche Ausrichtung von Schafteil und Kugel wird dann dadurch erzielt, dass das zusammengefügten Zwischenelementpaar auf den Schaftteil in derjenigen Ausrichtung aufgesetzt wird, dass der gewünschte räumliche Ausgleich geschaffen wird. Die Gelenkkugel ist dagegen symmetrisch geformt und kann in beliebiger Ausrichtung aufgesetzt werden.

Insbesondere geeignet ist das erfindungsgemäße Prothesensystem für den Einsatz als Hüftgelenkendoprothese, so dass sich auf diesen Anwendungsfall auch die Ausführungsbeispiele und große Teile der Beschreibung beziehen.

Je nach Anwendungsfall können das erste und das zweite Zwischenelement so ausgestaltet sein, dass sich der Offset - also die nicht-koaxiale Ausrichtung -bei entsprechender Ausrichtung entweder zu einem vollständigen koaxialen Fluchten oder zu einem minimalen Offset - also dem kleinsten bei der vorgesehenen Zwischenelementkombination vorgesehenen Nichtfluchten - verstellen lässt.

Die erfindungsgemässen - aus zwei Zwischenelementen bestehenden - Adapter lassen sich in unterschiedlichen Gesamtlängen erzeugen, so dass neben dem einstellbaren radialen Versatz auch eine axiale Anpassung an den beim jeweiligen Patienten vorliegenden Anwendungsfall erfolgen kann.

Eine sehr kompakte Bauform des Adapters des erfindungsgemäßen aus zwei Zwischenelementen bestehenden Adapters lässt sich (axialer Richtung) dann erreichen, wenn der Durchmesser des Innenkonus des ersten Zwischenelements den des zweiten Zwischenelements übertrifft und sich diese Konen beider Zwischenelemente sich in axialer Richtung im Wesentlichen überdecken.

Die minimale Ausdehnung in axialer Richtung des Adapters des erfindungsgemäßen modularen Gelenkprothesensystems ergibt sich, wenn die den Innenkonus aufweisenden Flächen des ersten und des zweiten Zwischenelements im zusammengefügten Zustand in einer Ebene gelegen sind.

Vorteilhaft ist es weiterhin, wenn bei dem erfindungsgemäßen modularen Gelenkprothesensystem bei sowohl einen radialen Versatz als auch eine Abwinklung aufweisenden Zwischenelementen beide Maßnahmen bei jedem der Zwischenelemente eine Verlagerung des betreffenden Konus radial in die selbe Richtung bewirken.

Weitere vorteilhafte Weiterbildungen ergeben sich aus den übrigen Unteransprüchen.

Wenn bei gleichzeitig vorgesehenem radialen Versatz und Abwinklung der Achsenrichtungen von Innen- und Außenkonus des ersten und des zweiten Zwischenelements diese so gewählt sind, dass die Achse des Außenkonus des ersten Zwischenelements in jeder eingestellten Position die Mittelachse des Innenkonus des zweiten Zwischenelements in seinem Fußbereich schneidet ergibt sich eine physiologische Neigung des Gelenkkopfes in Abhängigkeit von der Einbauausrichtung, da die Neigung dem radialen Versatz in der Weise folgt, dass der Kopf um so mehr geneigt ist, desto größer sein radialer Versatz ist.

Zur vereinfachten Bedienung ist ein Navigationssystem vorgesehen mit Mitteln um aus dem Computerromogramm vor dem Eingriff der zu behandelnden Seite des Patienten oder spiegelsymmetrisch aus der gegenüberliegenden Seite die Sollpositionen von Gelenkkugel und Gelenkpfanne zu ermitteln und während der Operation durch Vermessung von den aktuellen Positionen der Gelenkkugel und der Gelenkpfanne den Ausaleichsbedarf zu ermitteln und rechnerisch aus den zur Verfügung stehenden Zwischenelementen und deren Einstellmö2lichkeiten eine Auswahl zu treffen und anzuzeigen.

Ein vorteilhaftes Ausführungsbeispiel der Erfindung ist in den Figuren jeweils als Schnittdarstellung wiedergegeben. Es zeigen:
Fig. 1 eine schematische Übersicht einer modularen Prothese gemäß der Erfindung.
Fign. 2 und 3 eine entsprechende Prothese, bei der bei kurzer Bauform des Adapters bestehend aus dem ersten und dem zweiten Zwischenelement mit ausschließlich radialem Versatz diese in einer ersten und zweiten. Position für ein erstes und zweites Offset eingestellt sind.
Fign. 4 und 5 eine entsprechende Prothese, bei der bei langer Bauform des Adapters bestehend aus dem ersten und dem zweiten Zwischenelement mit ausschließlich radialem Versatz diese in einer ersten und zweiten Position für ein erstes und ein zweites Offset eingestellt sind.
Fign. 6 bis 9 eine entsprechende Prothese, bei der bei kurzer Bauform des Adapters bestehend aus dem ersten und dem zweiten Zwischenelement mit radialem Versatz in einem und einer Abwinklung in beiden Zwischenelementen diese in einer vier Position für vier unterschiedliche Offsets eingestellt sind.
Fign. 10 bis 13 eine entsprechende Prothese, bei der bei langer Bauform des Adapters des-ersten und des zweiten Zwischenelements mit radialem Versatz in einem und einer Abwinklung in beiden Zwischenelementen diese in einer vier Position für vier unterschiedliche Offsets eingestellt sind.
Fign. 14 und 15 eine entsprechende Prothese, bei der bei kurzer Bauform des Adapters des ersten und des zweiten Zwischenelements mit radialem Versatz und Abwicklung in beiden Zwischenelementen diese in zwei Position für zwei unterschiedliche Offsets eingestellt sind,
Fig. 16 eine Prothese bei der schematisch - und in diesem Fall räumlich - die mit verschiedenen Adapterlängen mit den erfindungsgemäßen Maßnahmen erzielbaren Positionen der Gelenkkugel in Bezug auf den Schaft dargestellt sind sowie
Fig. 17 in Draufsicht Markierungen zur Verwendung mit einem Navigationssystems gemäß der Erfindung..

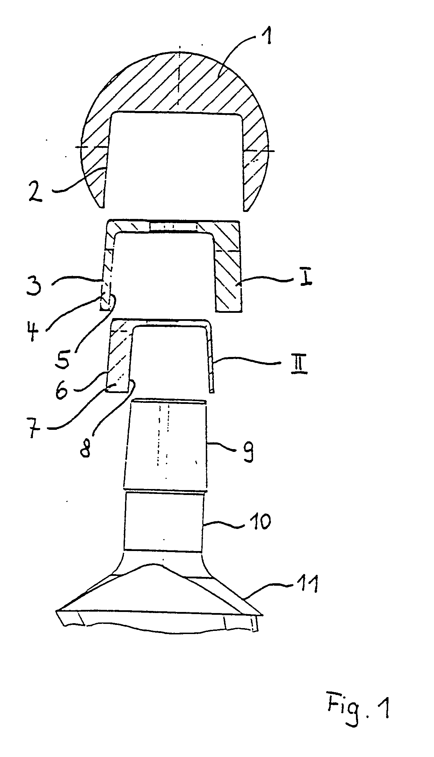

Bei der in Fig. 1 dargestellten schematischen Übersicht einer modularen Prothese gemäß der Erfindung sind die Einzelteile explodiert dargestellt, um die Bezeichnungen der einzelnen Elemente, welche in den folgenden Darstellungen wiederholt vorkommen, zu definieren. Da es sich im Prinzip immer wieder um dieselben Elemente handelt, welche nur jeweils andere Größen und Positionen aufweisen konnten die Bezugszeichen hier entfallen. Es handelt sich um das erfindungsgemäße modulare Gelenkprothesensystem, bestehend aus einem Prothesenschaft 11 mit einem Hals 10 und einem Außenkonus 9; einem ersten Zwischenelement 4, das zwischen dem Außenkonus 9 des Prothesenschaits 11 und dem Innenkonus 2 der Gelenkkugel 1 einfügbar ist, wobei das erste Zwischenelement 4 einen Außenkonus 3, der dem Innenkonus 2 der Gelenkkugel 1 angepasst ist und einen Innenkonus 5 aufweist, und wobei der Außenkonus 3 und der Innenkonus 5 des ersten Zwischenelements 4 nicht koaxial angeordnet sind, bei dem der Innenkonus 5 des ersten Zwischenelements 4 dem Außenkonus 6 eines zweiten Zwischenelements 7 angepasst ist, und das zweiten Zwischenelement einen Innenkonus 8 aufweist, der dem Außenkonus 9 des Prothesenhalses 10 angepasst ist; wobei die Achsen des Innenkonus 8 und des Außenkonus 6 des zweiten Zwischenelements 7 ebenfalls nicht koaxial angeordnet sind, und zwar derart dass der Außenkonus 3 und der Innenkonus 5 des ersten Zwischeneletnents 4 und der Außenkonus 6 und der Innenkonus 8 des zweiten Zwischenelements 7 unter sich jeweils um einen solchen Betrag radial versetzt und/oder in einem solchen Winkel angeordnet sind, dass sich bei zusammengefügten und entsprechend ausgerichteten erstem und zweitem Zwischenelement 4, 7, die Neigung und/oder der axiale Versatz der Achsenrichtungen des Außenkonus 3 des ersten 4 und des Inneakonus 8 des zweiten Zwischenelements 7 bis auf einen vorgegebenen Längenwert oder auch vollständig kompensieren, während in den anderen Positionen sich die entsprechenden rein Positionen sich die entsprechenden Neigungen und/oder radialen Versatze zu unterschiedlich einstellbaren Werten addieren.

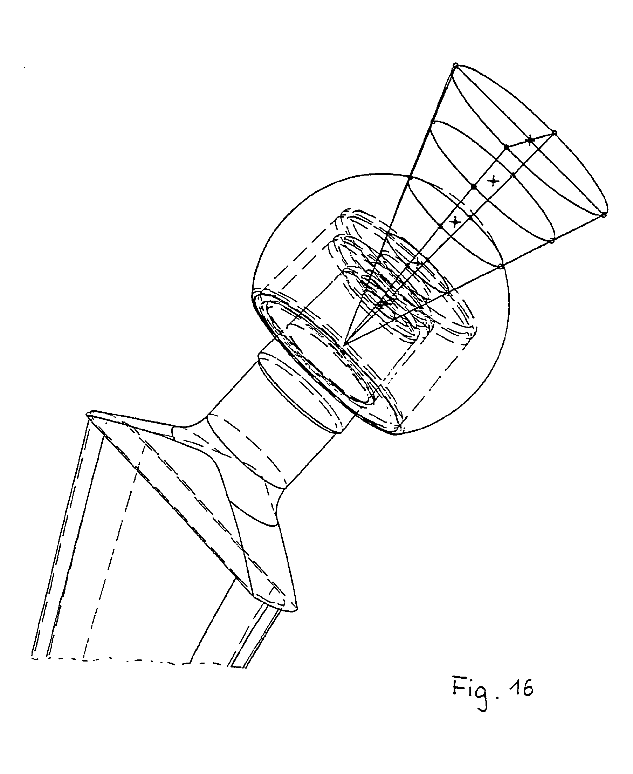

Die übrigen der Figuren sind insoweit selbsterklärend, als dass hier jeweils dargestellt wird, wie sich verschiedene Positionen der Gelenkkugel - und damit des Gelenkdrehpunkts - durch unterschiedliche Positionierung des ersten und zweiten Adapters erzielen lassen. Zusätzlich ist noch die Länge des zweiten Adapters variabel. Er kann in verschiedenen Längenstufungen ausgewählt werden, so dass ein großer räumlicher Bereich erreicht werden kann, wie aus der Darstellung gemäß Figur 16 hervorgeht. Der geometrisch dargestellte Kegel 12 zeigt mit seinen verschiedenen Stufungen, welche Bereiche mit den einzelnen Zwischenelementen erreicht werden können. In der Darstellung gemäß Figur 16 sind fünf Längenstufungen vorgesehen. Die Ebenen in den einzelnen Stufungen können mit den Maßnahmen gemäß der Erfindung jeweils stufenlos erreicht werden.

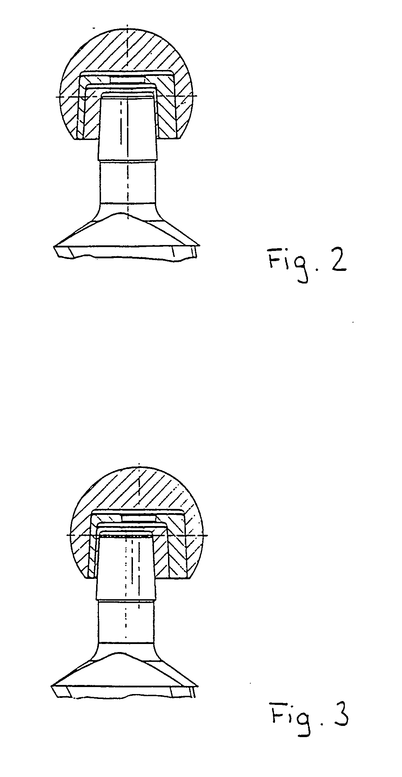

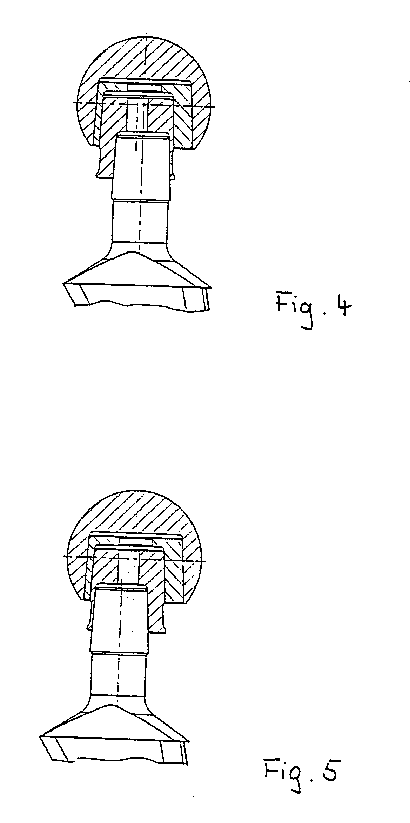

Bei den in den Fign. 1 bis 5 dargestellten Prothesen sind die Konen der Zwischenelemente gerade ausgerichtet, weisen also lediglich einen radialen Versatz auf.

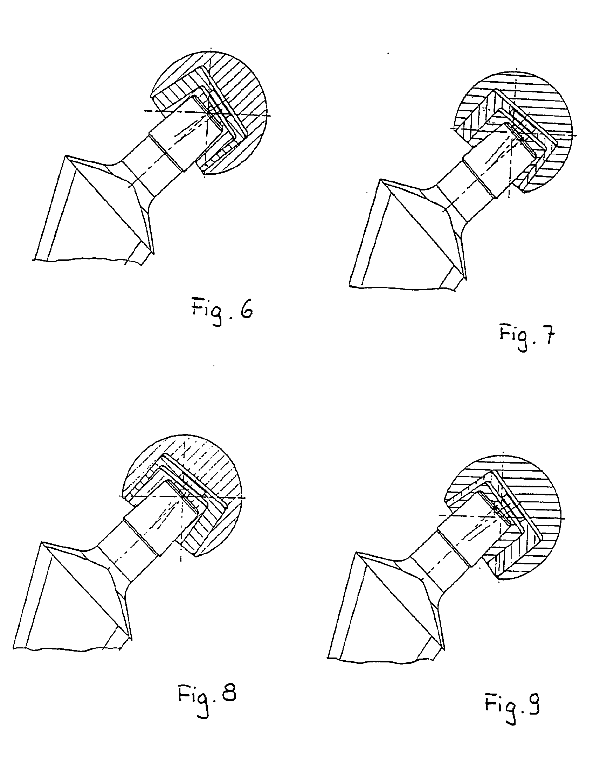

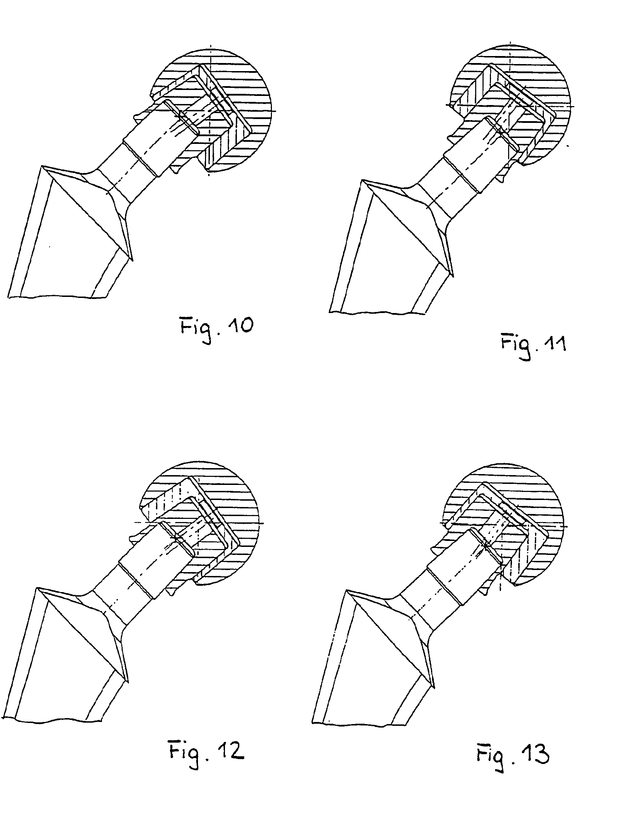

In den Figuren 6 bis 13 weisen die Achsen des Innen- und des Außenkonus des zweiten Zwischenelements eine relative Neigung zueinander auf, so dass sich mit der Auswahl der Einstellung auch die Ausrichtung der Gelenkkugel im Raum ändert. Durch diese Maßnahme lässt sich beim Anpassen der Dimensionen der Gelenkendoprothese an die Einbausituation auch die Ausrichtung der Gelenkpfanne berücksichtigen. Bei der Einstellung der Zwischenelemente wirken hier also axialer Versatz und Neigung wahlweise additiv oder subtraktiv zusammen, was bei der Ausrichtung berücksichtigt werden muss. Außerdem sind hier ebenfalls wieder unterschiedliche Längen des zweiten Zwischenelements zur Variation der axialen Abmessungen vorgesehen.

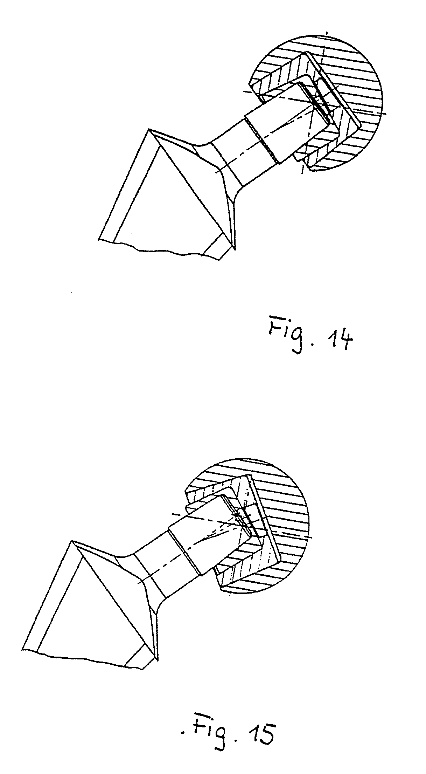

In den Ausführungsbeispielen gemäß der Figuren 14 und 15 sind der Innen- und der Außenkonus sowohl des Innen- als auch des Außenkonus relativ zueinander geneigt, so dass die Variationsmöglichkeit der Neigung der Achse der Gelenkkugel noch vergrößert ist. Auf diese Weise kann der Bewegungsraum (Range of Motion) bei Einbau maximiert werden.

Bei den größeren Längen des zweiten Zwischenelements ist eine umlaufende Einschnürung vorgesehen, welche ebenfalls den Bewegungsraum vergrößert.

Mit einem in den Zeichnungen nicht dargestellten Navigationsystem sind Mittel vorgesehen, um aus dem Computertomogramm vor dem Eingriff der zu behandelnden Seite des Patienten oder spiegelsymmetrisch aus der gegenüberliegenden Seite die Sollpositionen von Gelenkkugel und Gelenkpfanne zu ermitteln und während der Operation durch Vermessung von den aktuellen Positionen der Gelenkkugel und der Gelenkpfanne den Ausgleichsbedarf zu ermitteln und rechnerisch aus den zur Verfügung stehenden Zwischenelementen und deren Einstellmöglichkeiten eine Auswahl zu treffen und anzuzeigen.

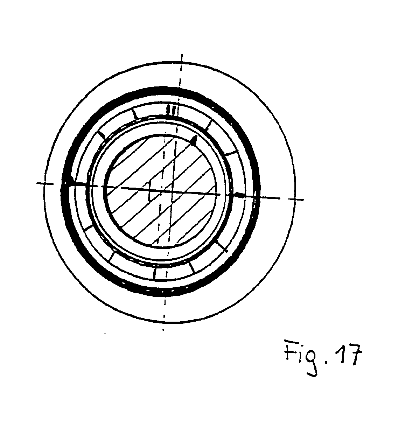

Die in Fig. 17 dargestellten Markierungen in Uhrzeitanordnung können vorteilhaft mit einem Navigationssystems gemäß der Erfindung in der Weise verwendet werden, als das durch das Navigationssystem unter Zugrundelegung einer im System abgespeicherten Nachschlagetabelle aus der gemessenen Geometrie automatisch Einstellwerte für das erste und zweite Zwischenelement ermittelt und angezeigt werden. Diese bilden bevorzugt Uhrzeitwerte, wobei auf dem zweiten Zwischenelement das Zifferblatt einer 12h Uhr wiedergegeben ist. Feste Markierungen auf dem zweiten Zwischenelement (innen) und dem Prothesenschaft (außen), bilden dann sozusagen, den kleinen und den großen Zeiger einer Uhr, so dass das Navigationssystem Uhrzeitwerte im 12h System ausgeben kann, die leicht zu memorieren und zu übertragen sind.

## Patentansprüche

1. Modulares Gelenkprothesensystem, bestehend aus einem Prothesenschaft (11) mit einem Hals (10) und einem Außenkonus (9), einem ersten Zwischenelement (4), das zwischen dem Außenkonus (9) des Prothesenschafts (11) und dem Innenkonus (2) der Gelenkkugel (1) einfügbar ist, wobei das erste Zwischenelement (4) einen Außenkonus (3), der dem Innenkonus (2) der Gelenkkugel (1) angepasst ist und einen Innenkonus (5( aufweist, und wobei der Außenkonus (3) und der Innenkonus (5) des ersten Zwischenelements (4) nicht koaxial angeordnet ist, **dadurch gekennzeichnet, dass** der Innenkonus (5) des ersten Zwischenelements (4) dem Außenkonus (6) eines zweiten Zwischenelements (7) angepasst ist, und das zweite Zwischenelement einen Innenkonus (8) aufweist, der dem Außenkonus (9) des Prothesenhalses (10) angepasst ist, wobei die Achsen des Innenkonus (8) und des Außenkonus (6) des zweiten Zwischenelements (7) ebenfalls nicht koaxial angeordnet sind, und zwar derart, dass der Außenkonus (3) und der Innenkonus (5) des ersten Zwischenelements (4) und der Außenkonus (6) und der Innenkonus (8) des zweiten Zwischenelements (7) unter sich jeweils um einen solchen Betrag radial versetzt und/oder in einem solchen Winkel angeordnet sind, dass sich bei zusammengefügten und entsprechend ausgerichteten erstem und zweitem Zwischenelement (4,7) die Neigung und/oder der axiale Versatz der Achsenrichtungen des Außenkonus (3) des ersten (4) und des Innenkonus (8) des zweiten Zwischenelements (7) bis auf einen vorgegebenen Längenwert oder auch vollständig kompensieren, während in den anderen Positionen sich die entsprechenden Neigungen und/oder radialen Versatze zu unterschiedlich einstellbaren Werten addieren.

2. Modulares Gelenkprothesensystem nach Anspruch 1, **dadurch gekennzeichnet, dass** das erste Zwischenelement aus Keramik besteht.

3. Modulares Gelenkprothesensystem nach Anspruch 1, **dadurch gekennzeichnet, dass** sich bei entsprechend ausgerichteten erstem und zweitem Zwischenelement die Neigung und/oder der axiale Versatz der Achsenrichtungen des ersten und des zweiten Zwischenelements bzw. der radiale Versatz der Achsrichtungen des Innenkonus der Gelenkkugel und des zweiten Zwischenelements vollständig kompensieren.

4. Modulares Gelenkprothesensystem nach einem der vorangehenden Ansprüche, **dadurch gekennzeichnet, dass** der Durchmesser des Innenkonus des ersten Zwischenelements den des zweiten Zwischenelements übertrifft und sich diese Konen beider Zwischenelemente in axialer Richtung im Wesentlichen überdecken.

5. Modulares Gelenkprothesensystem nach einem der vorangehenden Ansprüche, **dadurch gekennzeichnet, dass** die den Innenkonus aufweisenden Flächen des ersten und des zweiten Zwischenelements im zusammengefügten Zustand in einer Ebene gelegen sind.

6. Modulares Gelenkprothesensystem nach einem der vorangehenden Ansprüche, **dadurch gekennzeichnet, dass** bei sowohl einen radialen Versatz als auch eine Abwinklung aufweisenden Zwischenelementen beide Maßnahmen bei jedem der Zwischenelemente eine Verlagerung des betreffenden Konus radial in dieselbe Richtung bewirken.

7. Modulares Gelenkprothesensystem nach einem der vorangehenden Ansprüche, **dadurch gekennzeichnet, dass** bei gleichzeitig vorgesehenem radialen Versatz und Abwinklung der Achsenrichtungen von Innen- und Außenkonus des ersten und des zweiten Zwischenelements diese so gewählt sind, dass die Achse des Außenkonus des ersten Zwischenelements in jeder eingestellten Position die Mittenachse des Innenkonus des zweiten Zwischenelements in seinem Fußbereich schneidet.

8. Modulares Gelenkprothesensystem nach einem der vorangehenden Ansprüche, **dadurch gekennzeichnet, dass** das erste aus Metall bestehende Zwischenelement mit der aus Keramik bestehenden Gelenkkugel fest verbunden ist.

9. Modulares Gelenkprothesensystem nach einem der vorangehenden Ansprüche, **dadurch gekennzeichnet, dass** an einer dem Innenkonus benachbarten Fläche der aus Keramik bestehenden Gelenkkugel eine Ausnehmung vorgesehen ist, welche die im Material der Gelenkkugel auftretenden Spannungen vergleichmäßigt.

10. Modulares Gelenkprothesensystem nach einem der vorangehenden Anspruche, **dadurch gekennzeichnet, dass** bei einem System mit Zwischenelementen, bei dem unterschiedliche Längenstufungen auswählbar sind und bei dem die Achse des Innenkonus in Bezug auf den Außenkonus versetzt und/oder abgewinkelt ist, die Anzahl der angebotenen Winkelstufungsdifferenzen und/oder Versatzmaßdifferenzen der derart ausgewählt ist, dass die erreichbaren Radienstufungen der Abwinklung bzw. des Versatzes den angebotenen Längenstufungsdifferenzen entsprechen.

11. Modulares Gelenkprothesensystem nach einem der vorangehenden Ansprüche, **dadurch gekennzeichnet, dass** auf den im zusammengesetzten Zustand sichtbaren Stirnseiten des ersten und zweiten Zwischenelements Einstellmarkierungen vorgesehen sind.

12. Modulares Gelenkprothesensystem nach Anspruch 11, **dadurch gekennzeichnet, dass** es sich bei den Einstellmarkierungen um eine Grad- oder Stundeneinteilung nach Art eines Uhrenziffernblattes handelt, wobei am Schaft und am anderen Zwischenelement jeweils feste Markierungen nach Art eines Grad- oder Uhrzeigers vorgesehen sind.

13. Modulares Gelenkprothesensystem nach einem der vorangehenden Anspruch, **dadurch gekennzeichnet, dass** die am Schaft befindliche Markierung, den großen Zeiger und der am anderen Zwischenelement befindliche Zeiger den kleiner Zeiger einer Uhr bildet und die Einstellwerte für die Positionierung von der Recheneinheit in Uhrzeiten, entsprechend Stunden- und Minutenwerten, ausgegeben werden.

14. Modulares Gelenkprothesensystem nach einem der vorangehenden Ansprüche, **dadurch gekennzeichnet, dass** der Schaft des zweiten Zwischenelements die Basisfläche des ersten Zwischenelements überragt.

15. Modulares Gelenkprothesensystem nach einem der vorangehenden Anspruche, **dadurch gekennzeichnet, dass** der die Basisfläche des ersten Zwischenelements überragende Teil des zweiten Zwischenelements eine umlaufende Einschnürung aufweist, welche den Bewegungsraum des Prothesenschafts relativ zur Gelenkpfanne vergrößert.

16. Navigationssystem für ein modulares Gelenkprothesensystem nach einem der Ansprüche 11 bis 15, **dadurch gekennzeichnet, dass** zum Ermitteln der Positionierungen der miteinander zu verbindenden Gelenkteile eine nachgeschaltete Recheneinheit vorgesehen ist, welche unmittelbar Einstellwerte für die Auswahl und Positionierung der Zwischenelemente auf Grund von an dem Zwischenelement vorgesehenen Symbolen umrechnet.

17. Navigationssystem nach Anspruch 17, **dadurch gekennzeichnet, dass** Mittel vorgesehen sind, um aus dem Computertomogramm vor dem Eingriff der zu behandelnden Seite des Patienten oder spiegelsymmetrisch aus der gegenüberliegenden Seite die Sollpositionen von Gelenkkugel und Gelenkpfanne zu ermitteln und während der Operation durch Vermessung von den aktuellen Positionen der Gelenkkugel und der Gelenkpfanne den Ausgleichbedarf zu ermitteln und rechnerisch aus den zur Verfügung stehenden Zwischenelementen und deren Einstellmöglichkeiten eine Auswahl zu treffen und anzuzeigen.

## Claims

1. Modular joint prosthesis system consisting of a prosthesis shaft **(11)** with a neck **(10)** and an outer cone **(9),** a first intermediate element **(4)** which can be fitted between the outer cone **(9)** of the prosthesis shaft **(11)** and the inner cone **(2)** of the joint ball **(1),** wherein the first intermediate element **(4)** has an external cone **(3)** adjusted to the inner cone **(2)** of the joint ball **(1)** and an internal cone **(5)** and wherein the external cone **(3)** and the internal cone **(5)** of the first intermediate element **(4)** are not positioned coaxially, **characterized in that** the internal cone **(5)**of the first intermediate element **(4)** is adjusted to the external cone **(6)** of a second intermediate element **(7)** and the second intermediate element has an internal cone **(8)** which is adjusted to the outer cone **(9)** of the prosthesis shaft **(10),** wherein the axes of the internal cone **(8)** and the external cone **(6)** of the second intermediate element **(7)** are also not positioned coaxially, that is in such a way that the external cone **(3)** and the internal cone **(5)** of the first intermediate element **(4)** and the external cone **(6)** and the internal cone **(8)** of the second intermediate element **(7)** respectively among one another are positioned radially misaligned by such an amount and/or positioned at such an angle that the angularity and/or the axial misalignment of the directions of the axes of the external cone **(3)** of the first **(4)** and the internal cone **(8)** of the second intermediate element **(7)** are compensating each other up to a preset length measure or even fully, when the first and the second intermediate elements **(4, 7)** are fitted together and adequately aligned, whereas in the other positions the respective angularities and/or radial misalignments add up to differently adjustable values.

2. Modular joint prosthesis system according to claim 1, **characterized in that** the first intermediate element consists of ceramic material.

3. Modular joint prosthesis system according to claim 1, **characterized in that** the angularity and/or the axial misalignment of the axes directions of the first and the second intermediate elements respectively the radial misalignment of the axes directions of the inner cone of the joint ball and of the second intermediate element are fully compensating each other when the first and the second intermediate elements are adequately aligned.

4. Modular joint prosthesis system according to one of the preceding claims, **characterized in that** the diameter of the internal cone of the first intermediate element is bigger than that of the second intermediate element and that these cones of both intermediate elements in essence cover each other in axial direction.

5. Modular joint prosthesis system according to one of the preceding claims, **characterized in that** the surfaces constituting the internal cone of the first and the second intermediate elements in the assembled state are on one plane.

6. Modular joint prosthesis system according to one of the preceding claims, **characterized in that** at intermediate elements both having a radial misalignment and a slope, both proceedings at each of the intermediate elements cause a shift of the respective cone radially into the same direction.

7. Modular joint prosthesis system according to one of the preceding claims, **characterized in that** in case of simultaneously provided radial misalignment and slope of the axes directions of internal and external cones of the first and the second intermediate elements these are chosen that way, that the axis of the external cone of the first intermediate element in each selected position cuts the center axis of the internal cone of the second intermediate element in the region of its base.

8. Modular joint prosthesis system according to one of the preceding claims, **characterized in that** the first intermediate element consisting of metal is fixed to the joint ball consisting of ceramic material.

9. Modular joint prosthesis system according to one of the preceding claims, **characterized in that** in a surface of the ceramic joint ball adjacent to the inner cone is provided a recess which evens out the stress occurring in the material of the joint ball.

10. Modular joint prosthesis system according to one of the preceding claims, **characterized in that** in a system with intermediate elements in which different length dimensions can be chosen and in which the axis of the internal cone in relation to the external cone is offset and/or sloped the number of offered different angles and/or different dimensions of misalignment is chosen in such a way that the available different radii of the slope respectively the misalignment correspond with the offered different length dimensions.

11. Modular joint prosthesis system according to one of the preceding claims, **characterized in that** the visible in the assembled state front surfaces of the first and second intermediate elements are provided with alignment marks.

12. Modular joint prosthesis system according to claim 11, **characterized in that** the alignment marks are realized as graduated arc or hour circle like on the face of a clock, wherein the shaft and the other intermediate element are provided with fix marks of the kind of a graduation or time indicator.

13. Modular joint prosthesis system according to one of the preceding claims, **characterized in that** the marking provided on the shaft forms the minute hand and the indicator provided on the other intermediate element forms the hour hand of a clock and the setting values for the positioning are put out by the computer unit in times, that is in hours and minutes.

14. Modular joint prosthesis system according to one of the preceding claims, **characterized in that** the shaft of the second intermediate element projects over the base area of the first intermediate element.

15. Modular joint prosthesis system according to one of the preceding claims, **characterized in that** the portion of the second intermediate element projecting over the base area of the first intermediate element has a circumferential necking that enlarges the space for movements of the prosthesis shaft with regard to the joint socket.

16. Navigation system for a modular joint prosthesis system according to one of the claims 11 to 15, **characterized in that** for the determination of the positions of the joint parts to be assembled with each other is provided a subsequent computer unit which directly converts setting values for selecting and positioning the intermediate elements on the basis of symbols provided on the intermediate element.

17. Navigation system according to claim 16, **characterized in that** means are provided to determine the desired positions of joint ball and joint socket from the CT scan of the side of the patient needing therapy or mirror-symmetric of the opposite side prior to the operation and during the operation to determine the needed compensation and to mathematically select from the available intermediate elements and their positioning possibilities and to indicate them by measuring the current positions of the joint ball and the joint socket.

## Revendications

1. Système de prothèse d'articulation modulaire, constitué d'une tige de prothèse (11) pourvue d'un col (10) et d'un cône externe (9), d'un premier élément intermédiaire (4), insérable entre le cône externe (9) de la tige de prothèse (11) et le cône interne (2) de la sphère d'articulation (1), le premier élément intermédiaire (4) présentant un cône externe (3) qui est adapté au cône interne (2) de la sphère d'articulation (1), et un cône interne (5), le cône externe (3) et le cône Interne (5) du premier élément Intermédiaire (4) n'étant pas disposés coaxialement, **caractérisé en ce que** le cône interne (5) du premier élément Intermédiaire (4) est adapté au cône externe (6) d'un second élément intermédiaire (7) et **en ce que** le second élément Intermédiaire présente un cône Interne (8) qui est adapté au cône externe (9) du col de prothèse (10), les axes du cône interne (6) et du cône externe (6) du second élément intermédiaire (7) n'étant pas non plus disposés coaxialement, de telle sorte que le cône externe (3) et le cône interne (5) du premier élément intermédiaire (4) et le cône externe (6) et le cône interne (6) du second élément intermédiaire (7) sont respectivement radialement décalés entre eux d'une valeur telle et/ou disposés selon un angle tel que lorsque le premier et le second élément intermédiaire (4, 7) sont assemblés et alignés de manière adéquate, l'inclinaison et/ou le décalage axial des directions des axes du cône externe (3) du premier (4) et du cône Interne (8) du second élément intermédiaire (7) se compensent à une valeur de longueur prédéfinie près ou aussi totalement, alors que dans les autres positions les inclinaisons et/ou décalages radiaux correspondants s'additionnent des valeurs réglables différemment.

2. Système de prothèse d'articulation modulaire selon la revendication 1, **caractérisé en ce que** le premier élément intermédiaire est en céramique.

3. Système de prothèse d'articulation modulaire selon la revendication 1, **caractérisé en ce que** lorsque le premier et le second élément intermédiaire sont orientés de manière adéquate, l'inclinaison et/ou le décalage axial des directions des axes du premier et du second élément intermédiaire, respectivement le décalage radial des directions des axes du cône Interne de la sphère d'articulation et du second élément intermédiaire se compensent totalement.

4. Système de prothèse d'articulation modulaire selon l'une des revendications précédentes, **caractérisé en ce que** le diamètre du cône Interne du premier élément intermédiaire dépasse celui du second élément intermédiaire et **en ce que** lesdits cônes des deux éléments Intermédiaires se recouvrent essentiellement en direction axiale.

5. Système de prothèse d'articulation modulaire selon l'une des revendications précédentes, **caractérisé en ce que** les surfaces du premier et du second élément Intermédiaire présentant le cône interne sont à l'état assemblé situées dans un seul plan.

6. Système de prothèse d'articulation modulaire selon l'une des revendications précédentes, **caractérisé en ce que** dans le cas où les éléments intermédiaires présentent à la fois un décalage radial et une forme angulaire, les deux mesures conduisent dans chacun des éléments intermédiaires à un déplacement radial du cône concerné dans la même direction.

7. Système de prothèse d'articulation modulaire selon l'une des revendications précédentes, **caractérisé en ce qu'**en présence à la fois d'un décalage radial et d'une forme angulaire des directions des axes des cônes interne et externe du premier et du second élément Intermédiaire, ceux-cl sont tels que dans toute position réglée l'axe du cône externe du premier élément Intermédiaire coupe l'axe médian du cône interne du second élément intermédiaire au niveau de sa base.

8. Système de prothèse d'articulation modulaire selon l'une des revendications précédentes, **caractérisé en ce que** le premier élément intermédiaire en métal est solidaire de la sphère d'articulation en céramique.

9. Système de prothèse d'articulation modulaire selon l'une des revendications précédentes, **caractérisé en ce qu'**un creux est prévu sur une surface voisine du cône interne de la sphère d'articulation en céramique, lequel creux homogénéise les contraintes qui apparaissent dans le matériau de la sphère d'articulation.

10. Système de prothèse d'articulation modulaire selon l'une des revendications précédentes, **caractérisé en ce que** dans un système pourvu d'éléments intermédiaires, dans lequel différentes graduations de longueurs peuvent être sélectionnées et dans lequel l'axe du cône interne est décalé et/ou présente une forme angulaire par rapport au cône externe, le nombre des différences de graduations angulaires et/ou différences de cotes de décalage proposés est tel que les graduations de rayons possibles de la forme angulaire resp. du décalage correspondent aux différences de graduations de longueurs proposées.

11. Système de prothèse d'articulation modulaire selon l'une des revendications précédentes, **caractérisé en ce que** des repères de réglage sont prévus sur les faces visibles à l'état assemblé du premier et du second élément intermédiaire.

12. Système de prothèse d'articulation modulaire selon la revendication 11, **caractérisé en ce que** les repères de réglage sont une graduation en degrés ou en heures de type cadran de montre, des repères fixes de type indicateur des degrés ou aiguille de montre étant prévus sur la tige et sur l'autre élément intermédiaire respectivement.

13. Système de prothèse d'articulation modulaire selon l'une des revendications précédentes, **caractérisé en ce que** le repère situé sur la tige constitue la grande aiguille et l'aiguille située sur l'autre élément intermédiaire la petite aiguille d'une montre et **en ce que** les valeurs de réglage pour le positionnement sont fournies en temps par l'unité de calcul, selon des heures et des minutes.

14. Système de prothèse d'articulation modulaire selon l'une des revendications précédentes, **caractérisé en ce que** la tige du second élément intermédiaire fait saillie de la surface de base du premier élément intermédiaire.

15. Système de prothèse d'articulation modulaire selon l'une des revendications précédentes, **caractérisé en ce que** la partie du second élément intermédiaire, qui fait saillie de la surface de base du premier élément intermédiaire, présente une contraction périphérique qui augmente l'espace de mouvement de la tige de prothèse par rapport à la cavité cotyloïde.

16. Système de navigation pour un système de prothèse d'articulation modulaire selon l'une des revendications 11 à 15, **caractérisé en ce que** pour déterminer les positionnements des pièces d'articulation à relier, une unité de calcul placée en aval est prévue, qui recalcule instantanément les valeurs de réglage pour la sélection et le positionnement des éléments Intermédiaires sur la base de symboles prévus sur l'élément intermédiaire.

17. Système de navigation selon la revendication 17, **caractérisé en ce que** des moyens sont prévus, permettant à partir de la tomodensitométrie réalisée avant l'intervention du côté à traiter du patient, ou symétriquement à partir du côté opposé, de déterminer les positions désirées de la sphère d'articulation et de la cavité cotyloïde et pendant l'opération, par des mesures, de déterminer le besoin de compensation par rapport aux positions actuelles de la sphère d'articulation et de la cavité cotyloïde, puis de procéder par calcul à une sélection parmi les éléments intermédiaires disponibles et leurs possibilités de réglage, et d'indiquer celle-ci.
